# EUROPÄISCHE PATENTANMELDUNG

(11) **EP 2 896 597 A1**
(43) Veröffentlichungstag der Anmeldung: **22.07.2015**
(21) Anmeldenummer: 14151884.5
(22) Anmeldetag: 21.01.2014
(51) Int. Cl.: C01B 31/28

(54) **Verfahren zum An- und Abfahren eines Phosgengenerators**

(71) Anmelder: Bayer MaterialScience AG, 51373 Leverkusen (DE)
(72) Erfinder: Die Erfindernennung liegt noch nicht vor
(74) Vertreter: BIP Patents

(57) **Zusammenfassung**

Die vorliegende Erfindung betrifft ein Verfahren zum Betreiben eines Phosgengenerators zur Herstellung von Phosgen durch Umsetzung von Kohlenmonoxid mit Chlor in der Gasphase an einem Aktivkohle-Katalysator, der in einem Reaktionsraum angeordnet ist, bei dem die Phosgenherstellung nach einer vorgebbaren Betriebsdauer durch Abfahren des Phosgengenerators über eine Abfahrdauer zumindest zeitweise unterbrochen und nach einer vorgebbaren Stillstandzeit durch Anfahren des Phosgengenerators über eine Anfahrdauer wieder aufgenommen wird, wobei das Verfahren dadurch gekennzeichnet ist, dass der Aktivkohle-Katalysator vor dem Anfahren des Phosgengenerators durch Zugabe von Kohlenmonoxid soweit von Chlor befreit wird, dass während der Anfahrdauer eine Höchstkonzentration von Chlor im Gasstrom unmittelbar stromabwärts des Reaktionsraumes von 1000 ppmv nicht überschritten wird. Die Erfindung betrifft zudem die Verwendung des so gewonnenen Phosgens in der Herstellung von Polycarbonat und Isocyanaten.

## Beschreibung

Die vorliegende Erfindung betrifft ein Verfahren zum Betreiben eines Phosgengenerators zur Herstellung von Phosgen durch Umsetzung von Kohlenmonoxid mit Chlor in der Gasphase an einem Aktivkohle-Katalysator, der in einem Reaktionsraum angeordnet ist, bei dem die Phosgenherstellung nach einer vorgebbaren Betriebsdauer durch Abfahren des Phosgengenerators über eine Abfahrdauer zumindest zeitweise unterbrochen und nach einer vorgebbaren Stillstandzeit durch Anfahren des Phosgengenerators über eine Anfahrzeit wieder aufgenommen wird.

Phosgen findet in vielen Bereichen der Chemie Anwendung, sei es als Hilfsstoff oder als Zwischenprodukt. Das mengenmäßig größte Einsatzgebiet stellt die Herstellung von Diisocyanaten als Ausgangsstoffe für die Polyurethanchemie dar. Insbesondere sind hier die Stoffe 2,4- und 2,6-Toluylendiisocyanat (TDI), die Isomere und Homologen von Diphenylmethandiisocyanat (MDI) und Hexamethylendiisocyanat (HDI) zu nennen.

Die großtechnische Herstellung von Phosgen aus Kohlenmonoxid und Chlor ist aus dem Stand der Technik bekannt (z.B. Ullmann's Encyclopedia of Industrial Chemistry, 5th ed. Vol. A 19p 413f., VCH Verlagsgesellschaft mbH, Weinheim, 1991). Dabei wird Kohlenmonoxid im stöchiometrischen Überschuss mit Chlor vereinigt und über einen Festbettkatalysator geleitet. Als Katalysator wird für industrielle Zwecke Aktivkohle eingesetzt, wobei die Auswahl einer geeigneten Aktivkohle bis heute empirisch erfolgt (Mitchell et al.: Selection of carbon catalysts for the industrial manufacture ofphosgene; Catal. Sci. Technol., 2012, 2, 2109-2115).

Die Aktivkohle verbraucht sich mit der Zeit und muss regelmäßig erneuert werden. Die Möglichkeiten der Regenerierung des Aktivkohlekatalysators wurde von E. Wygasch in ,Ullmann's Encyklopädie der Technischen Chemie', (Urban & Schwarzenberg, München, 13 (1962) 493) vorgeschlagen, wobei die Reaktivierung der Aktivkohle bei Temperaturen von 550 °C bis 630 °C vorgenommen wird.

Für die Phosgenherstellung werden aus Gründen der Anlagensicherheit und aus Gründen der Produktqualität hohe Anforderungen an die Reinheit der Einsatzstoffe Kohlenmonoxid und Chlor gestellt. Die Einsatzstoffe sollten niedrige Gehalte an Methan und Wasserstoff aufweisen, da diese während der Vereinigung mit Chlor zu einer stark exothermen Reaktion führen können. Der Temperaturanstieg kann dabei zu einer gefährlichen Reaktion zwischen Chlor und dem Apparatewerkstoff, dem sogenannten Chlor-Eisen-Brand führen.

Die Einsatzstoffe sollten niedrige Gehalte an Schwefel, Brom und Iod aufweisen, da diese im erzeugten Phosgen verbleiben können und beim Einsatz des Phosgens in einem nachgelagerten Prozess, wie beispielsweise der Herstellung von Isocyanaten, zu Qualitätseinbußen führen können. Solche Qualitätseinbußen sind beispielsweise eine schlechtere Farbe des Endproduktes.

Aus dem Stand der Technik sind Verfahren zur Herstellung von Phosgen bekannt, das einen geringen Gehalt an Nebenprodukten aufweist. Beispielsweise werden durch eine geeignete Verfahrensführung Gehalte von weniger als 150 ppm an Tetrachlorkohlenstoff erreicht (EP 1 135 329 B1), oder es wird Chlor als Einsatzstoff gefordert, das weniger als 50 ppm (EP 118 78 08 B1), bzw. weniger als 400 ppm (EP 152 90 33 B1) an freiem oder gebundenem Brom oder Iod enthält.

Aus der EP 1 808 430 B1 ist ein Verfahren zur Herstellung von Isocyanaten bekannt, bei dem Phosgen eingesetzt wird, das weniger als 100 ppm Schwefel in elementarer oder gebundener Form enthält.

Die Bildung von Phosgen ist eine stark exotherme Reaktion mit einer Bildungsenthalpie von -107,6 kJ/mol. Gebildetes Phosgen unterliegt einem Dissoziationsgleichgewicht und zerfällt bei erhöhten Temperaturen wieder zu den Ausgangsstoffen. Bei 100 °C dissoziiert Phosgen bereits so weit, dass es ca. 50 ppm Chlor enthält.

Restgehalte an Chlor im erzeugten Phosgen sind bei praktisch allen Verwendungsmöglichkeiten dieses Zwischenproduktes störend. Um den Chlorgehalt des erzeugten Phosgens, so niedrig wie möglich zu halten, wird einerseits Kohlenmonoxid im Überschuss eingesetzt und andererseits die Phosgenbildungsreaktion bei möglichst niedrigen Temperaturen vervollständigt.

Bei der Herstellung von Phosgen wird daher Kohlenmonoxid mit einen Überschuss von 3 Vol-% bis 10 Vol-% der stöchiometrisch nötigen Menge eingesetzt. Das im Überschuss eingesetzt Kohlenmonoxid kann nicht rezykliert werden und stellt einen materiellen Verlust dar. Aufgrund dessen wurden in der Vergangenheit vielfache Anstrengungen unternommen, wirtschaftlichere Verfahren zur Herstellung von Phosgen zu entwickeln. So ist in EP 2 067 742 A1 ein Verfahren zur Herstellung von Phosgen bei verminderter CO-Emission bzw. verminderten CO-Verlusten durch eine Hauptvereinigung, eine nachfolgende Kondensation des Phosgens und einer anschließenden Nachvereinigung des Restgases mit Chlor beschrieben. Ein Verfahren mit Regelkonzept zur Minimierung des CO-Überschusses stellt WO 2010/103029 A1 vor.

Ein weiterer wesentlicher Aspekt bei der Herstellung von Phosgen ist die sichere und gleichmäßige Abfuhr der Reaktionswärme. Dies wird beispielsweise erreicht, in dem die Reaktion in einem Rohrbündelreaktor - dem sogenannten Phosgengenerator - durchgeführt wird, in dessen Rohren sich die Aktivkohle befindet und um dessen Reaktionsrohre ein Kühlmedium in erzwungener Konvektion oder in natürlicher Konvektion geführt und dort teilverdampft wird. In EP 0 134 506 B1 ist ein Verfahren beschrieben, in dem die Kühlung genutzt werden kann, um Dampf zu erzeugen. In solchen Rohrbündelreaktoren läuft die Phosgenbildungsreaktion zwischen Kohlenmonoxid und Chlor an geeigneten Aktivkohle-Katalysatoren bereits bei ca. 40 °C - 50 °C ab, wobei die Temperatur in der Katalysatorschüttung in den Rohren auf bis zu ca. 600 °C ansteigen kann und - je nach Intensität der angewandten Kühlung - bis zum Reaktoraustritt wieder auf 40 °C - 150 °C absinkt.

Es ist ferner bekannt, das Katalysatorbett auf unter 100 °C abzukühlen, da dann Phosgen mit Restgehalten an Chlor von weniger als 50 ppm erhalten werden kann. Für viele Anwendungsgebiete, wie zum Beispiel die Isocyanaterzeugung für die Polyurethanherstellung, stellt ein solcher Chlorgehalt bereits die Obergrenze der Spezifikation dar, da es sonst zu Qualitätseinbußen kommen kann. Dies ist beispielsweise in EP 0 134 506 B1 beschrieben. Solche Qualitätseinbußen sind beispielsweise ein erhöhter Gehalt an chlorierten Nebenprodukten oder eine schlechtere Farbe des Endproduktes.

Um derartige Qualitätseinbußen bei der Isocyanaterzeugung zu vermeiden, ist es somit erforderlich sowohl den stöchiometrischen Überschuss an Kohlenmonoxid als auch erhöhte Reaktoraustrittstemperaturen am Phosgengenerator jederzeit zu vermeiden.

Den beschriebenen Verfahren des Standes der Technik gelingt es zwar, ein Phosgen herzustellen, das bei den Endprodukten zu keinen Qualitätseinbußen führt, jedoch werden nur Verfahren beschrieben, die sich im Normalbetrieb befinden. Anfahrprozesse bis zum Erreichen eines stabilen Betriebszustandes bei der angestrebten Soll-Last (sogenannte "Anfahrzeit") oder Abfahrprozesse bis zum Erreichen der kompletten Abschaltung (sogenannte "Abfahrzeit") werden nicht berücksichtigt. Untersuchungen, die zu der vorliegenden Erfindung geführt haben, haben jedoch gezeigt, dass während der An- und Abfahrzeit erhöhte Chlormengen entstehen können, die in den Produktstrom gelangen.

Die Aufgabe der vorliegenden Erfindung besteht somit darin, ein Verfahren der eingangs genannten Art zur Verfügung zu stellen, das bereits in der Anfahrzeit die Erzeugung von Phosgen mit einem geringen Gehalt an Chlor, erlaubt. Vorzugsweise soll das Verfahren es gestatten, Phosgen mit einem Gehalt an Chlor von höchstens 100 ppmv am Austritt des Phosgengenerators zu erzeugen.

Diese Aufgabe wird gelöst durch ein Verfahren zum Betreiben eines Phosgengenerators zur Herstellung von Phosgen durch Umsetzung von Kohlenmonoxid mit Chlor in der Gasphase an einem Aktivkohle-Katalysator, der in einem Reaktionsraum angeordnet ist, bei dem die Phosgenherstellung nach einer vorgebbaren Betriebsdauer durch Abfahren des Phosgengenerators über eine Abfahrdauer zumindest zeitweise unterbrochen und nach einer vorgebbaren Stillstandzeit durch Anfahren des Phosgengenerators über eine Anfahrzeit wieder aufgenommen wird, wobei das Verfahren dadurch gekennzeichnet ist, dass der Aktivkohle-Katalysator vor dem Anfahren des Phosgengenerators durch Zugabe von Kohlenmonoxid soweit von Chlor befreit wird, dass während der Anfahrzeit eine Höchstkonzentration von Chlor im Gasstrom unmittelbar stromabwärts des Reaktionsraumes von 1000 ppmv nicht überschritten wird.

Der vorliegenden Erfindung liegt die Erkenntnis zugrunde, dass das Verdrängen von in dem Aktivkohle-Katalysator und die ihn umgebende Reaktorperipherie befindlichen Chlors in ausreichender Weise dadurch erfolgen kann, dass der Phosgengenerator mit Kohlenmonoxid durchspült (beschleiert) wird und somit der Aktivkohle-Katalysator auf geeignete Weise behandelt wird. Es wird also der Aktivkohle-Katalysator und die ihn umgebende Reaktorperipherie vor dem Anfahren des Prozesses von Chlor befreit, bevor im Zuge des Anfahrens Kohlenmonoxid und vor allem Chlor wieder in den Phosgengenerator geleitet werden. Dabei wird insbesondere auch das Chlor auf geeignete Weise verdrängt, das an der Aktivkohle gebunden ist. Die Behandlung der Aktivkohle kann beispielsweise a) vor der Anfahrzeit und/oder b) während der Abfahrzeit und/oder c) nach der Abfahrzeit durchgeführt werden. Diese Methoden sind auf unterschiedliche Weise realisierbar, die im Folgenden im Detail erörtert werden.

Im Rahmen der vorliegenden Erfindung bedeutet die Einheit "ppmv" parts per million by volume und bezieht sich dabei auf eine Temperatur von 298,15 K und einen Druck von 1013 hPa. Die Volumenkonzentration an Chlor in einem Phosgen haltigen Gasstrom kann beispielsweise UV-spektrometrisch bestimmt werden.

Die Einheit Normkubikmeter beschreibt diejenige Gasmenge, die unter festgelegten Bedingungen (Temperatur, Druck, Luftfeuchtigkeit) ein Gasvolumen von einem Kubikmeter einnehmen würde. Im Rahmen der vorliegenden Erfindung ist der Normkubikmeter auf einen Druck von 1013,25 hPa, einer Luftfeuchtigkeit von 0 % (trockenes Gas) und einer Temperatur von 273,15 K (tn = 0 °C) (Normbedingungen nach DIN 1343, STPD) bezogen.

Das zur Umsetzung verwendete Chlor kann nach üblichen technischen Verfahren wie Chloralkali- oder Chlorwasserstoffelektrolyse hergestellt werden und sollte möglichst rein sein. Besonders geeignet ist Chlor mit einem Reinheitsgrad von mehr als 98%. Vorzugsweise wird flüssiges Chlor aus einem Lagerbehälter verwendet, das in einem beheizten Vergaser verdampft und anschließend in einem Nachverdampfer von eventuell mitgerissenem, flüssigem Chlor befreit wird.

Das für die Umsetzung verwendete Kohlenmonoxid kann nach üblichen Verfahren, beispielsweise aus Erdgas/Naphtha in einer Synthesegasanlage oder aus Koks durch Blasen mit Sauerstoff hergestellt werden. Es hat sich als besonders vorteilhaft erwiesen, wenn das Kohlenmonoxid einen Methangehalt von weniger als 50 ppm aufweist.

Als Kohlenstoffkatalysator wird bevorzugt Aktivkohle eingesetzt. Vorzugsweise wird als Katalysator körnige Aktivkohle mit einem Korndurchmesser von ca. 3 bis 10, vorzugsweise 3,5 bis 7 mm benutzt. Die Porenoberfläche der Aktivkohle beträgt vorzugsweise etwa 1000 m²/g. Das Schüttgewicht der verwendeten Aktivkohle beträgt vorzugsweise etwa 450 g/l. Als Aktivkohle kommen insbesondere solche Typen in Betracht, die eine Druckhärte von mehr als 18 kp (bestimmt gemäß der Methode in DE-AS 2 322 706, Kolonne 6, Zeilen 28 - 38) und ein Benzol-Aufnahmevermögen von über 40 Gew.-% aufweisen. Gut geeignet sind beispielsweise die, diesen Bedingungen entsprechenden, bruch- und abriebfesten Aktivkohlen hoher Temperaturstandfestigkeit gemäß DE-AS 2 322 706.

An- und Abfahrzeiten treten bei der Phosgenerzeugung im industriellen Alltag häufig auf und sind nicht zwangsläufig mit einem Öffnen oder einem anderen mechanischen Eingriff in den Phosgengenerator verbunden, sondern nur mit dem Abstellen und erneuten Starten der Phosgenproduktion. Diese An- und Abfahrzeiten zeichnen sich in der Praxis dadurch aus, dass es zu Abweichungen im Kohlenmonoxidüberschuss und zu Restgehalten von Chlor im Phosgen kommen kann. Dies ist insbesondere dann zu beobachten wenn der Gasstrom sehr klein ist im Vergleich zum Gasstrom bei Volllast. Diese Restgehalte an Chlor sind nachteilig, da für den nachfolgenden Einsatz, insbesondere zur Herstellung von Isocyanaten, Chlorrestgehalte von max. 50 ppmv gefordert werden.

Nach einer bevorzugten Weiterbildung des erfindungsgemäßen Verfahrens wird eine Höchstkonzentration von Chlor im Gasstrom unmittelbar stromabwärts des Reaktionsraumes von 100 ppmv, insbesondere 50 ppmv nicht überschritten, wobei bevorzugt eine Konzentration von 10 ppmv nicht überschritten wird.

In bevorzugter Ausgestaltung des erfindungsgemäßen Verfahrens wird zur Einhaltung der Höchstkonzentration von Chlor während des Abfahrens des Phosgengenerators die Chlorzufuhr über die Abfahrdauer reduziert oder unmittelbar unterbrochen, wobei die Zufuhr an Kohlenmonoxid so lange aufrechterhalten wird, bis die Höchstkonzentration von Chlor erreicht oder unterschritten ist. Dies kann ein Zeitraum von mehreren Stunden sein, beispielsweise 3 oder 5 Stunden. Auf diese Weise kann verhindert werden, dass während des Abfahrens eine erhöhte Chlorkonzentration im Phosgenstrom auftritt. Ebenso wird eine Chloranreicherung im Generator verhindert, die bei einem erneuten Anfahren zu einer erhöhten Chlorkonzentration im Produktstrom führen könnte.

Hierbei kann der Aktivkohlekatalysator während der Abfahrdauer auf einer Temperatur von 60 °C bis 140 °C gehalten werden, wodurch die Reaktionsgeschwindigkeit von Chlor mit Kohlenmonoxid auf einem hohen Niveau gehalten wird.

Alternativ oder zusätzlich zu der vorgenannten Verfahrensvariante kann auch bei einem bereits im Stillstand befindlichen Phosgenreaktor verhindert werden, dass sich während des Anfahrens eine erhöhte Chlorkonzentration im Phosgenstrom einstellt. Dabei befindet sich der Phosgengenerator in der Stillstandzeit, wobei vor dessen Anfahren die Kohlenmonoxid Zufuhr gestartet sowie
a) der Aktivkohle-Katalysator auf eine Temperatur von 60 °C bis 140 °C erwärmt und/ oder
b) der Kohlenmonoxid Gasstrom auf eine Temperatur von 130 °C bis 250 °C erwärmt wird,
sodass das noch aus dem vorigen Produktionszyklus auf dem Aktivkohle-Katalysator und/ oder im Reaktionsraum vorhandene Chlor bis zum Erreichen oder Unterschreiten der Höchstkonzentration abreagiert.

Nach einer besonders bevorzugten Ausführungsform des erfindungsgemäßen Verfahrens beträgt die zum Erreichen oder Unterschreiten der Höchstkonzentration an Chlor eingesetzte Menge an Kohlenmonoxid mindestens 40 Normkubikmeter pro Tonne Aktivkohle-Katalysator im Reaktionsraum, insbesondere mindestens 60 Normkubikmeter pro Tonne, bevorzugt mindestens 80 Normkubikmeter pro Tonne.

Im Rahmen der vorliegenden Erfindung kann der Beginn des Anfahrens des Phosgengenerators durch den Start der Chlorzufuhr definiert sein. Um sicherzustellen, dass der Katalysator eine ausreichende Aktivität besitzt, bevor die Chlorzufuhr gestartet wird, kann der Aktivkohle-Katalysator auf eine Temperatur von wenigstens 140 °C erwärmt werden, insbesondere wenigstens 180 °C erwärmt werden.

Die Chlorzufuhr kann über die Anfahrzeit bis zu einem gewünschten Endwert gesteigert werden, wobei die Steigerung insbesondere stufenweise erfolgt und die Stufen vorzugsweise 25%, 50%, 75% und 100% des gewünschten Endwerts betragen und/ oder wobei die stufenweise Steigerung bevorzugt in gleichen Zeitintervallen erfolgt. Dem Fachmann ist bekannt, dass ein kontinuierlich betriebener industrieller Prozess ausgehend von einer nicht in Betrieb befindlichen Produktionsanlage (z. B. nach kurzzeitigem Stillstand) nicht augenblicklich wieder auf die Prozessparameter vor dem Produktionsstillstand hochgefahren werden kann. Edukte und Apparaturen müssen aufgeheizt werden, Apparate müssen ggf. inertisiert werden, die Belastung der Apparate mit den Edukten wird allmählich auf den angestrebten Soll-Wert gesteigert. Wenn eine Produktionsanlage zur Herstellung von Phosgen bei einer Soll-Belastung M'Soll von x [m³(Chlor)/h] betrieben werden soll, so kann diese Soll-Belastung beispielsweise erreicht werden, indem zu Beginn der Phosgenherstellung die Belastung M' zunächst auf einen Wert von 0,25 x eingestellt und die Belastung dann über die Zwischenstufen M' = 0,50 x und M' = 0,75 x innerhalb von 4 Stunden auf den Wert M' = x = M'Soll gesteigert wird. Alternativ kann auch von einem bestimmten Startwert aus, z. B. M' = 0,50 x, eine kontinuierliche Laststeigerung bis M' = x durchgeführt werden. M'Soll der Phosgenherstellung richtet sich nach dem Verbraucher des Phosgens, da immer ein kleiner Phosgenbestand in den Anlagen angestrebt wird. Diese Beispiele stehen natürlich nur exemplarisch für eine Vielzahl möglicher Anfahrprozeduren, deren genaue Ausgestaltung von den konkreten Gegebenheiten einer Produktionsanlage abhängt und daher nicht verallgemeinert werden kann. Ein gemeinsames Merkmal aller denkbaren Anfahrprozeduren ist jedoch, dass erst nach Verstreichen eines Zeitraums t die angestrebte Soll-Belastung von x erreicht wird. Dieser Zeitraum t wird erfindungsgemäß als Anfahrzeit bezeichnet. Durch die kontrollierte Steigerung der Chlorzufuhr über die Anfahrzeit können Spitzenwerte hinsichtlich der Chlorkonzentration vermieden werden, die möglicherweise nicht rechtzeitig abreagieren können und damit sonst in den Produktstrom gelangen könnten.

In weiter bevorzugter Weise kann bei dem erfindungsgemäßen Verfahren während der Anfahrzeit und der Betriebsdauer ein molarer Überschuss an CO gegenüber Chlor von 2 Mol-% bis 20 Mol-% eingestellt werden, insbesondere 3 Mol-% bis 15 Mol-%.

Ein weiterer Gegenstand der vorliegenden Erfindung ist die Verwendung von nach dem erfindungsgemäßen Verfahren gewonnenem Phosgen in der Herstellung von Polycarbonat und Isocyanaten. Dieses Phosgen kann aufgrund des geringen Gehaltes an Chlor für die Isocyanatherstellung ohne vorherige Aufbereitung verwendet werden, insbesondere ohne einen separaten Chlorentfernungsschritt.

Die eigentliche Herstellung von Phosgen kann grundsätzlich nach allen aus dem Stand der Technik bekannten Verfahren durchgeführt werden. Für die Ausführung dieses Schritts des erfindungsgemäßen Verfahrens kann ein 'Kaltvereiniger' entsprechend EP 1 640 341 B1 und 'Heißvereiniger'entsprechend EP 0 134 506 B1 benutzt werden. Die Inhalte dieser Patente sind per Verweis vollständig in die vorliegende Anmeldung aufgenommen.

Bei der Kaltvereinigung wird zur Herstellung von Phosgen Kohlenmonoxid und Chlor in Gegenwart von elementarem Kohlenstoff bei einer Temperatur von 30 °C bis 80 °C und einem Druck von 120 kPa abs. bis 400 kPa abs., gemessen unmittelbar hinter dem Phosgengenerator, umgesetzt. Bei diesem Verfahren können übliche Rohrreaktoren aus Normalstahl oder Edelstahl verwendet werden, dessen Rohre mit dem Kohlenstoffkatalysator gefüllt sind. Der Rohrreaktor kann dabei kontinuierlich oder diskontinuierlich betrieben werden.

Kohlenmonoxid und Chlor werden zu etwa gleichen Teilen, beispielsweise bei Raumtemperatur, in den Reaktor eingeführt. Um sicherzustellen, dass das gesamte Chlor umgesetzt wird, kann ein geringer Überschuss von Kohlenmonoxid verwendet werden. Vorzugsweise werden beide Reaktanden vor Eintritt in den Reaktor in einer geeigneten Mischvorrichtung, wie einem statischen Mischer, vermischt. Bei diesem Verfahren ist es von Vorteil, dass keine spezielle Vorbereitung des Katalysators erforderlich ist.

Der aus dem Reaktor austretende Gasstrom sollte eine Temperatur von 80 °C, gemessen unmittelbar hinter dem Phosgengenerator, nicht überschreiten, wobei die Temperatur des aus dem Reaktor austretenden Gasstroms vorzugsweise im Bereich von 40 °C bis 70 °C liegt. Zur Einhaltung dieser gewünschten Temperaturen kann eine entsprechende Kühlvorrichtung vorgesehen sein, mit welcher die bei der Reaktion frei werdende Reaktionswärme abgeführt und ein Überhitzen des Katalysators verhindert wird.

Der Druck unmittelbar stromabwärts des Phosgengenerators beträgt vorzugsweise höchstens 300 kPas abs. Auf diese Weise wird sichergestellt, dass kein Phosgen im Reaktor kondensiert.

Das am Kopf des Reaktors austretende Phosgen wird vorzugsweise bei Temperaturen von -10 °C bis -20 °C kondensiert.

Bei der sogenannten Heißvereinigung (beschrieben beispielsweise in EP 0 134 506 B1) wird Phosgen durch Umsetzung von Chlor mit Kohlenmonoxid in Aktivkohle als Katalysator-enthaltenen Rohrreaktoren unter gleichzeitiger Nutzung der anfallenden Reaktionswärme zur Erzeugung von Dampf umgesetzt. Dabei wird in einem ersten, gekörnte Aktivkohle enthaltenen Rohrreaktor mit einem lichten Rohrdurchmesser von maximal 100 mm 95 Vol.-% bis 98 Vol.-% des eingesetzten Chlors mit überschüssigem Kohlenmonoxid bei Reaktionstemperaturen von über 250 °C zu Phosgen umgesetzt. Die hierbei anfallende Reaktionswärme wird durch Verdampfungskühlung einer bei 150 °C bis 320 °C siedenden Flüssigkeit oder mit einer nicht-siedenden Flüssigkeit abgeführt, deren Temperatur am Reaktoraustritt mittels Zwangsumlaufpumpen und Temperatursteuerung bei 150 °C bis 320 °C gehalten wird. Der den Reaktor verlassende flüssige oder dampfförmige Wärmeträger wird in einem mit Wasser als Kühlmedium beschickten Wärmetauscher unter Erzeugung von Dampf kondensiert und / oder auf eine unter der Temperatur des Wärmeträgers am Reaktoraustritt liegende Temperatur abgekühlt und in den Reaktor zurückgeführt. Die den Reaktor verlassenden Reaktionsgase werden auf eine Temperatur von 50 °C bis 120 °C abgekühlt und anschließend in einen gekörnte Aktivkohle enthaltenen zweiten Reaktor geleitet, dessen Temperatur auf 50 °C bis 100 °C thermostatisch eingestellt und in dem die Umsetzung zu Ende geführt wird, so dass das den zweiten Reaktor verlassende Phosgen einen Restchlorgehalt von weniger als 50 ppmv aufweist. Die anschließende Kondensation des Phosgens erfolgt auf die bereits vorstehend beschriebene Weise.

Das erfindungsgemäße Verfahren kann beispielsweise in folgender Weise betrieben werden:
a) Chlor und Kohlenmonoxid werden in Gegenwart eines Aktivkohle-Katalysators in einem Rohrbündelreaktor enthaltend mehrere Reaktionsrohre und einen die Reaktionsrohre umgebenden Kühlmittelraum umgesetzt, wobei Chlor und Kohlenmonoxid mit einem molaren Überschuss an CO von 2 % bis 20 % eingesetzt werden, wobei anschließend die Menge M' des dem Phosgengenerator pro Stunde zugeführten Chlor-haltigen Stroms (a.1) innerhalb eines Zeitraums t₁ von Beginn der Phosgenherstellung bis zum Erreichen eines vorgegebenen Soll-Werts für M' gesteigert wird;
b) die in Schritt a) anfallende Reaktionswärme wird durch Verdampfungskühlung einer bei 150 °C bis 320 °C siedenden Flüssigkeit oder mit einer nicht siedenden Flüssigkeit abgeführt, deren Temperatur am Reaktoraustritt mittels Zwangsumlaufpumpen und Temperatursteuerung bei 150 °C - 320 °C gehalten wird;
c) die in Schritt a) erhaltenen Reaktionsgase werden beim Verlassen des Reaktors (1) auf eine Temperatur von 50 °C bis 120 °C abkühlt und
d) in einem, gekörnte Aktivkohle enthaltenden, zweiten Reaktor (2) geleitet, dessen Temperatur auf 50 °C bis 100 °C thermostatisiert wird und in dem die Umsetzung zu Ende geführt wird, so dass das den zweiten Reaktor verlassende Phosgen einen Restchlorgehalt von unter 100 ppmv aufweist.

Dies geschieht indem mindestens während eines Zeitraums to vor dem Beginn des Anfahrprozesses der Phosgenherstellung dem Reaktor (1) ein Kohlenmonoxidstrom (a.2) zugeführt wird, der vorhandenes Chlor verdrängt oder verbraucht.

Im Rahmen der vorliegenden Erfindung soll der Gehalt an Chlor, frei im Reaktorraum oder gebunden auf dem Aktivkohle-Katalysator, vor dem eigentlichen Anfahren - das mit der Zugabe von Chlor beginnt - auf einen möglichst niedrigen Wert gebracht werden. Dieses Ziel kann auf alternative Weisen erreicht werden, beispielsweise:
a) Es wird vor Beginn eines neuen Produktionszyklus, d. h. vor der Zufuhr von Chlor (a.1) und Kohlenmonoxid (a.2), die Anlage unter Beschleierung mit Kohlenmonoxid (a.2) ohne Chlor (a.1) mit Hilfe einer technischen Heizung auf Betriebstemperatur gebracht. Frühestens sobald der Phosgengenerator die gewünschte Temperatur von mindestens 140 °C, bevorzugt von mindestens 180 °C, erreicht hat, wird durch Zugabe von Chlor die Phosgenerzeugung gestartet.
b) Es werden nach Beendigung eines Produktionszyklus, d. h. nach Beendigung der Zufuhr von Chlor (a.1) und vollständiger Umsetzung der noch in der Produktionsanlage befindlichen Restmengen zu Phosgen der Kohlenmonoxidstrom (a.2) auf einen kleineren Strom reduziert, und für eine gewisse Zeitdauer aufrechterhalten, bevor endgültig der Prozess zur Herstellung von Phosgen abgestellt wird. Bei der Beschleierung mit Kohlenmonoxid wird die Restwärme der technischen Heizung und des Aktivkohle-Katalysators ausgenutzt. Somit minimiert sich die noch nach der Abstellung der kontinuierlichen Reaktion an der Aktivkohle anhaftende Chlormenge und kann beim Anfahren die Stöchiometrie zwischen Kohlenmonoxid und Chlor nicht stören.
c) Es wird ein abgestellter Phosgengenerator ausschließlich mit Kohlenmonoxid beschleiert. Dabei kann entweder der Phosgenerzeuger mittels technischer Heizung erhitzt werden, oder der Kohlenmonoxid-Strom vor Eintritt in den Phosgenerzeuger erhitzt werden, oder beides.

Allen Ausführungsformen ist gemein, dass sie den Gehalt an Chlor, frei im Reaktorraum oder gebunden auf dem Aktivkohle-Katalysator, reduzieren. Der Grad der Reduzierung, der durch die jeweilige Ausführungsform erreicht wird, hängt insbesondere von vier Faktoren ab:
- Kohlenmonoxid-Menge während der Beschleierung
- Temperatur des Kühlmantels des Phosgengenerators während der Beschleierung
- Temperatur des Kohlenmonoxids während der Beschleierung
- Dauer der Beschleierung

Die Steigerung eines oder mehrerer der vier Faktoren führt zu einer stärkeren Reduzierung des Gehalts an freiem oder gebundenem Chlor im Phosgengenerator.

Die Kohlenmonoxid-Menge zur Beschleierung kann beispielsweise 1 m³/h bis 250 m³/h betragen, bevorzugt 5 m³/h bis 100 m³/h, besonders bevorzugt 10 m³/h bis 50 m³/h.

Die Temperatur des Kühlmantels des Phosgengenerators während der Beschleierung kann 20 °C bis 250 °C betragen, bevorzugt 60 °C bis 200 °C, besonders bevorzugt 160 °C bis 200 °C.

Die Temperatur des Kohlenmonoxids während der Beschleierung kann 20 °C bis 250 °C betragen, bevorzugt 130 °C bis 250 °C, besonders bevorzugt 220 °C bis 250 °C.

Die Dauer der Beschleierung beträgt vorzugsweise mindestens 0,5 Stunden, bevorzugt mindestens 2 Stunden, besonders bevorzugt mindestens 6 Stunden. Auch Beschleierungsdauern von mehreren Tagen können im Sinne der vorliegenden Erfindung angewandt werden und vorteilhaft sein. Die Dauer der Beschleierung und Kohlenmonoxidmenge sind bei dieser Ausführungsform so zu kombinieren, dass bezogen auf die Masse des Aktivkohle-Katalysators mindestens 40 Normkubikmeter CO pro Tonne Aktivkohle-Katalysator zum Einsatz kommen, bevorzugt 60 Normkubikmeter und besonders bevorzugt 80 Normkubikmeter.

Durch geeignete Kombination der oben genannten Faktoren lassen sich auch positive Effekte erzielen, wenn die Aktivkohle sehr belastet war, oder wenn an dem Phosgengenerator schon einmal ein Chlordurchbruch registriert wurde.

Durch die erfindungsgemäßen Vorgehensweisen ergeben sich unter anderem die folgenden Vorteile für den Anfahrzeitraum der Herstellung von Phosgen:
i) Es steht für Kohlenmonoxid als Reaktionspartner nur das Chlor zur Verfügung, das beim Anfahren in den Phosgengenerator gefahren wird. Somit bleibt der gewünschte Überschuss an Kohlenmonoxid gegenüber Chlor bestehen.
ii) Die Temperaturkontrolle im Phosgengenerator bleibt in vollem Umfang präzise gewährleistet. Somit wird eine lokale thermische Überbelastung des Aktivkohlekatalysators vermieden. Dies erhöht dessen Standzeit.
iii) Vermeidung zu hoher Gehalte an freiem Chlor im Phosgen. Somit kommt es im nachfolgenden Einsatz des Phosgens zu einer reduzierten Bildung von chlorierten Verunreinigungen (z.B. Chlorierung von Lösungsmittel oder Isocyanat). Im Falle der Isocyanatherstellung führt dies zu einer Farbverbesserung.

Somit ermöglicht das erfindungsgemäße Verfahren durch einen niedrigen Gehalt von Chlor im Phosgengenerator vor dem eigentlichen Start der Phosgenerzeugung das Anfahren des Phosgengenerators und die anschließende Weiterverwendung des entstandenen Phosgens in technisch reibungsloser Weise ohne Ausfallzeiten mit direkt hoher Endproduktqualität der angestrebten Produkte, wie z.B. Isocyanate.

Das nach dem erfindungsgemäßen Verfahren hergestellte, am Kopf des Reaktors austretende Phosgen wird vorzugsweise bei Temperaturen von -10 °C bis -45 °C kondensiert. Es kann unmittelbar ohne weitere Reinigung zur Herstellung von Polycarbonaten oder zur Herstellung von Isocyanaten wie Methylendiphenyldiamin, Polymethylenpolyphenylpolyamin, Gemische aus Methylendiphenyldiamin und Polymethylenpolyphenylpolyamin, Toluylendiamin, Xylylendiamin, Hexamethylendiamin, Isophorondiamin und Naphthyldiamin, bevorzugt sind Methylendiphenyldiamin, Gemische aus Methylendiphenyldiamin und Polymethylenpolyphenylpolyamin sowie Toluylendiamin, oder zur Herstellung von pharmazeutischen Wirkstoffen oder zur Herstellung von Wirkstoffen für den Pflanzenschutz verwendet werden.

Die vorliegende Erfindung wird im Folgenden anhand von Ausführungsbeispielen näher erläutert.

### Beispiele

Gehaltsangaben in ppmv oder % sind Volumenanteile bezogen auf das Gesamtvolumen des jeweiligen Stoff(strom)s. Analysenwerte der Chlorgehalte wurden, sofern nicht anders angegeben, mittels UV-Spektrometrie bestimmt.

### Spezifikationen des eingesetzten Kohlenmonoxid und Chlor:

Kohlenmonoxid sollte eine Reinheit von mindestens 96 Vol.-% aufweisen, und Chlor eine Reinheit von mindestens 98 Vol.-%, wobei jeweils die folgenden Gehalte an Verunreinigungen nicht überschritten werden sollten:
In Kohlenmonoxid:

| Prüfmerkmal | | Sollwert | Maßeinheit |
|---|---|---|---|
| Kohlenmonoxid | (CO) | min. 96,0 | Vol.-% |
| Ammoniak | (NH₃) | max.100 | mg/Nm³ |
| Ges. Schwefel | (S) | max. 10 | mg/Nm³ |
| Methan | (CH₄) | max. 500 | Vol-ppm |
| Sauerstoff | (O₂) | max. 0,5 | Vol. % |
| Wasser | (H₂O) | max. 100 | Vol-ppm |
| Wasserstoff | (H₂) | max. 2 | Vol.-% |

In Chlor

| Prüfmerkmal | | Sollwert | Maßeinheit |
|---|---|---|---|
| Chlor | (Cl₂) | min. 98,0 | Vol.-% |
| Kohlendioxid | (CO₂) | max. 1 | Vol.-% |
| Sauerstoff | (O₂) | max. 1 | Vol.-% |
| Stickstoff | (N₂) | max. 1 | Vol.-% |
| Wasser | (H₂O | max. 64 | mg/Nm³ |

### Allgemeine Bedingungen für die Herstellung von Phosgen bei "eingefahrener" Produktionsanlage (d.h. nach Verstreichen der Anfahrzeit t) für Kaltvereiniger

In einem Mischrohr werden kontinuierlich 810 Nm³/h Chlor und 955 Nm³/h Kohlenmonoxid bei 19 °C und einem Druck von 1,8 bar absolut gemischt. Es wird ein Überschuss an Kohlenmonoxid, bezogen auf Chlor, eingesetzt, sodass nach der vollständigen Umsetzung des Chlors noch 9 % Kohlenmonoxid im Phosgen verbleiben. Das Mischgas aus Chlor und Kohlenmonoxid wird in einen am Boden befindlichen Verteiler eines Rohrbündel-Phosgengenerators gefahren. In den Rohren über dem Verteiler befinden sich als Katalysator 2 Tonnen Aktivkohle (Norit RB4C). An diesem Katalysator reagiert das Mischgas in einer stark exothermen Reaktion zu Phosgen ab. Die Reaktion wird über einen Wasserkreislauf mittels Wassersiedekühlung gekühlt. Die Temperatur des Phosgens in der Austrittsleitung des Generators beträgt 55 °C und der Druck liegt bei 1,53 bar absolut. An diesem Punkt wird die Vollständigkeit der Reaktion überwacht, indem der Restchlorgehalt und der Kohlenmonoxidgehalt kontinuierlich gemessen werden. Das so hergestellte, gasförmige, überschüssiges Kohlenmonoxid enthaltende Phosgen wird dann in einem Phosgenverflüssiger bei -17 °C kondensiert. Das Sumpfprodukt aus dem Phosgenverflüssiger läuft in einen Phosgenlösungstank. Überschüssiges Kohlenmonoxid kondensiert nicht und wird über Kopf in einen nachgeschalteten baugleichen zweiten Phosgengenerator gefahren und dort mit einer entsprechenden Menge an Chlor beaufschlagt, sodass wiederum nach vollständiger Umsetzung des Chlors noch 9 % an Kohlenmonoxid im Phosgen verbleiben. Auch nach dem zweiten Phosgengenerator wird die Vollständigkeit der Reaktion überwacht, indem der Restchlorgehalt und der Kohlenmonoxidgehalt kontinuierlich gemessen wird. Das so hergestellte Phosgen wird in einem zweiten Phosgenverflüssiger bei -17 °C kondensiert. Das Sumpfprodukt aus dem zweiten Phosgenverflüssiger läuft ebenfalls in den Phosgenlösungstank. Als Kopfprodukt wird überschüssiges Kohlenmonoxid, dem auch Spuren an Phosgen anhaften, in eine Abgasschiene geleitet, dort von Phosgen befreit und anschließend in einer thermischen Abluftreinigung verbrannt. Somit kommen pro Stunde 4,2 Tonnen Phosgen im Phosgenlösungstank an.

Im Phosgenlösungstank kann das Phosgen bei Bedarf mit einem Lösungsmittel vermischt werden. Für einen Herstellungsprozess für Isocyanate wird das Phosgen im Phosgenlösungstank mit Monochlorbenzol zu einer 35 %igen Phosgenlösung vermischt und bei -2 °C zur weiteren Verwendung, wie der Herstellung von Isocyanaten, entnommen. In anderen großtechnischen Verfahren kann es auch als reines Flüssigphosgen zum Einsatz kommen.

### Allgemeine Bedingungen für das Anfahren eines Phosgengenerators

Um die Nennkapazität der Anlage zu erreichen (M'_{Soll}), wird zunächst mit geringeren Mengenströmen an Chlor und Kohlenmonoxid gestartet (in den Beispielen 1 und 2 wird die Anlage mit 50 bis 60 % der Nennkapazität gestartet, was einer Produktionsleistung von ca. 2,5 t/h (Phosgen) entspricht).

Nach der Anfahrphase werden diese Mengenströme bis auf die gewünschte Solllast erhöht. Das Hochfahren der Produktionslast kann manuell oder mit einer Anfahrautomatik gestaltet werden. Die Anlage wird jeweils so schnell wie möglich auf die gewünschte Solllast hochgefahren, wobei das während der Anfahrphase entstehende Phosgen am Austritt des Phosgengenerators einen Restchlorgehalt von < 100 ppm an Chlor hat.

### Beispiel 1 (Vergleichsbeispiel): gemeinsame Zugabe von Chlor und CO führt zu 400 bzw. 1000 ppm Chlor in Phosgen.

Nach Reparaturarbeiten in der Phosgen-Anlage wird der Phosgengenerator angefahren, indem in ein mit Stickstoff inertisiertes Mischrohr gleichzeitig Chlor und Kohlenmonoxid gefahren werden. Die Menge der beiden Einsatzstoffe, die während der Anfahrzeit t von 45 Minuten, in das Mischrohr eingeleitet werden, wird stufenlos von 0 m³/h auf 545 Nm³/h Kohlenmonoxid und stufenlos von 0 m³/h auf 455 Nm³/h Chlor, erhöht. Nach 45 Minuten ergibt sich im Phosgen ein Gehalt an Kohlenmonoxid von 9 %. Die Temperatur im Mischrohr beträgt 18 °C, und der Druck stellt sich unmittelbar auf 1,8 bar absolut ein. Das Mischgas aus Chlor und Kohlenmonoxid tritt anschließend in den 18 °C warmen Innenraum des Rohrbündel-Phosgengenerator ein und verdrängt den inerten Stickstoff. Die Reaktion zu Phosgen springt direkt an und ist stark exotherm. Die Reaktionswärme wird über einen Wasserkreislauf mittels Wassersiedekühlung abgeführt. Die Temperatur des Phosgens in der Austrittsleitung des Generators liegt nach 5 Minuten bei 55 °C und der Druck liegt bei 1,53 bar absolut. An dieser Stelle hat das Phosgen während der Anfahrphase einen Restchlorgehalt > 100 ppm und in der Spitze > 1000 ppm (oberhalb des Messbereiches von 1000 ppm). Das so hergestellte Phosgen wird, wie in den allgemeinen Herstellungsbedingungen beschrieben, kondensiert und im Phosgenlösungstank gesammelt. Nach 45 Minuten werden diese Mengenströme auf die gewünschte Solllast erhöht. Das Hochfahren der Anlage kann manuell oder mit einer Anfahrautomatik gestaltet werden. Die Anlage wird jeweils so schnell wie möglich auf die Solllast hochgefahren.

Im Phosgenlösungstank kommt während der Anfahrphase Phosgen mit erhöhtem Chlorgehalt an, da das Chlor teilweise im Phosgenverflüssiger mitkondensiert. Die Menge an Chlor in Phosgen verdünnt sich kontinuierlich durch nachgeschobenes Phosgen im Laufe eines Tages auf < 50 ppm Chlor im Endprodukt Phosgen. Der Einsatz von diesem mit Chlor verunreinigtem Phosgen führt für 12 Stunden zu Farbproblemen bei der Phosgenierung von MDA zum Endprodukt MDI. Die Farbzahl des MDI liegt bei > 0,210 und in der Spitze bei 0,240 (Gelbwert).

Die Farbzahl (Gelbwert) wurde in der Weise bestimmt, dass von dem erhaltenen Isocyanat 1,0 g in Chlorbenzol gelöst und mit Chlorbenzol zu 50 ml verdünnt wurde. Von dieser Lösung wird bei einer Wellenlänge von 430 nm und einer Schichtdicke von 10 mm bei Raumtemperatur die Extinktion gegen Chlorbenzol bestimmt. Diese Methode wurde bei sämtlichen Angaben bezüglich des Gelbwertes zugrunde gelegt.

### Beispiel 2 (erfindungsgemäß) Vorlauf mit CO bevor die Phosgenerzeugung mit Zugabe von Chlor gestartet wird.

Eine Phosgen-Anlage wird angefahren, indem in ein inertisiertes Mischrohr um 1 Minute zeitversetzt Kohlenmonoxid und Chlor gefahren werden. Die Menge der beiden Einsatzstoffe, die während der Anfahrzeit t von 45 Minuten, für Chlor entsprechend nur 44 Minuten, in das Mischrohr eingeleitet werden, wird stufenlos von 0 m³/h auf 545 Nm³/h Kohlenmonoxid und stufenlos von 0 m³/h auf 455 Nm³/h Chlor, hochgezogen. Nach 45 Minuten ergibt sich im Phosgen ein Gehalt an Kohlenmonoxid von 9 %. Die Temperatur im Mischrohr beträgt 18 °C und der Druck stellt sich unmittelbar auf 1,8 bar absolut ein. Kohlenmonoxid und zeitversetzt das Mischgas aus Chlor und Kohlenmonoxid tritt in den 18 °C warmen Innenraum des Rohrbündel-Phosgengenerator ein und verdrängt den inerten Stickstoff. Die Reaktion zu Phosgen springt direkt an und ist stark exotherm. Die Reaktionswärme wird über einen Wasserkreislauf mittels Wassersiedekühlung abgeführt. Die Temperatur des Phosgens in der Austrittsleitung des Generators liegt nach 5 Minuten bei 55 °C und der Druck liegt bei 1,57 bar absolut. Das so hergestellte Phosgen wird, wie in den allgemeinen Herstellungsbedingungen beschrieben, kondensiert und im Phosgenlösungstank gesammelt. Nach 45 Minuten werden diese Mengenströme auf die gewünschte Solllast erhöht. Das Hochfahren der Anlage kann manuell oder mit einer Anfahrautomatik gestaltet werden. Die Anlage wird jeweils so schnell wie möglich auf die Solllast hochgefahren, wobei das während der Anfahrphase entstehende Phosgen einen Restchlorgehalt von 22 ppm hat, wobei dieser Wert in der Austrittsleitung des Generators ermittelt wird.

Der Einsatz von diesem Phosgen, das nur mit Spuren an Chlor verunreinigt ist, in der Phosgenierung von MDA führt zu einem annähernd farblosen Endprodukt MDI mit einer Farbzahl von 0,160 (Gelbwert).

### Allgemeine Bedingungen für die Herstellung von Phosgen bei eingefahrener Produktionsanlage (also nach Verstreichen der Anfahrzeit t₁) für Heißvereiniger

In einem Mischrohr werden kontinuierlich 3200 Nm³/h Chlor und 4230 Nm³/h Kohlenmonoxid bei 20 °C und einem Druck von 3,2 bar absolut gemischt. Es wird ein Überschuss an Kohlenmonoxid, bezogen auf Chlor, eingesetzt, so dass nach der vollständigen Umsetzung des Chlors noch 12 % Kohlenmonoxid im Phosgen verbleiben. Das Mischgas aus Chlor und Kohlenmonoxid wird in einen am Boden befindlichen Verteiler eines Rohrbündel-Phosgengenerators gefahren. In den Rohren über dem Verteiler befinden sich als Katalysator 4 Tonnen Aktivkohle (Norit RB4C). Der Katalysator ist durch einen Siebboden über und unterhalb des Rohrbodens gegen Austrag und gegen Herunterrieseln geschützt. An diesem Katalysator reagiert das Mischgas in einer stark exothermen Reaktion zu Phosgen ab. Die Reaktionswärme wird mittels Siedekühlung an ein Wärmeträgeröl (Dekalin) abgegeben, so dass die Temperatur des Phosgens in der Austrittsleitung des Generators 234 °C beträgt. Der Druck liegt bei 2,55 bar absolut. Aufgrund der hohen Reaktionstemperatur im Heißvereiniger kommt es zu einer Disproportionierung, so dass im Austrittsgasstrom noch 1-3% Chlor vorhanden sind.

Im Austritt des nachgeschalteten Gaskühlers wird die Vollständigkeit der Reaktion überwacht, indem der Restchlorgehalt und der Gehalt an Kohlenmonoxid kontinuierlich gemessen werden. Das so hergestellte, gasförmige, überschüssiges Kohlenmonoxid und eine geringe Menge Chlor enthaltende Phosgen wird dann in einem Gaskühler auf 117°C heruntergekühlt und in einen weiteren Rohrbündel-Phosgengenerator gefahren. In diesem zweiten Reaktor wird das sich im Gasstrom befindliche Chlor an einem Katalysator (Aktivkohle: Norit RB4C) zu Phosgen umgesetzt. Die Reaktion wird mittels Siedekühlung eines Wärmeträgers (Methylenchlorid) gekühlt. Die Temperatur des Phosgens in der Austrittsleitung des zweiten Generators beträgt 98 °C und der Druck liegt bei 2,46 bar absolut. Im Austritt des 2. Phosgenreaktors wird die Vollständigkeit der Reaktion ebenfalls überwacht, indem der Restchlorgehalt und der Gehalt an Kohlenmonoxid kontinuierlich gemessen werden.

Das so hergestellte chlorfreie Phosgen wird in einem Phosgenverflüssiger bei -17 °C kondensiert. Das Sumpfprodukt aus dem Phosgenverflüssiger läuft in die Phosgenvorlage der Phosgenabsorptionskolonne ab. Überschüssiges Kohlenmonoxid kondensiert nicht und wird über Kopf in einem Mischrohr mit einer entsprechenden Menge an Chlor beaufschlagt, danach in einen nachgeschalteten baugleichen dritten Phosgengenerator (Nachvereiniger) an Aktivkohle zu Phosgen umgesetzt. Die Reaktion wird mittels Siedekühlung eines Wärmeträgers (Methylenchlorid) gekühlt.

Nach vollständiger Umsetzung des zudosierten Chlors verbleiben noch 2,5 % an Kohlenmonoxid im Phosgen. Auch nach dem dritten Phosgengenerator wird die Vollständigkeit der Reaktion überwacht, indem der Restchlorgehalt und der Gehalt an Kohlenmonoxid kontinuierlich gemessen werden. Das so hergestellte Phosgen wird in einem Brüdenkondensator zusammen mit Phosgenbrüden aus der Phosgenierung bei -17 °C teilweise kondensiert und in die Phosgenvorlage der Phosgenabsorptionskolonne abgeführt. Das überschüssige Kohlenmonoxid wird zusammen mit dem in der Phosgenierung gebildeten HCl-Gas als Kopfprodukt der Phosgenabsorption in die HCl-Absorption geleitet. In einer der HCl-Absorption nachgeschalteten Brüdenkondensation wird Wasserdampf und HCl-Gas kondensiert, so dass als Restgas, das überschüssige Kohlenmonoxid, dem auch Spuren an Phosgen anhaften, verbleibt. Dieser Gasstrom wird in eine Abgasschiene geleitet, dort von Phosgen befreit und anschließend in einer thermischen Abluftreinigung verbrannt.

Somit werden pro Stunde 18 Tonnen Phosgen hergestellt und in der Phosgenabsorption mit einem Lösungsmittel vermischt. Für einen Herstellungsprozess für Isocyanate wird das Phosgen zu einer 45%igen Phosgenlösung in der Phosgenabsortion mit Monochlorbenzol vermischt und bei -7 °C zur weiteren Verwendung, wie der Herstellung von Isocyanaten, entnommen. In anderen großtechnischen Verfahren kann es auch als reines Flüssigphosgen zum Einsatz kommen.

### Beispiel 3 (Vergleichsbeispiel) Heißfahrweise: beim Aufheizen der Generatoren kein Vorlauf an Kohlenmonoxid.

Nach Reparaturarbeiten in der Phosgen-Anlage wird der Phosgengenerator angefahren, indem in ein mit Stickstoff inertisiertes Mischrohr, das ein Volumen von 100 Litern hat, gleichzeitig Chlor und Kohlenmonoxid gefahren werden. Die Menge der beiden Einsatzstoffe, die während der Anfahrzeit t von 30 Minuten, in das Mischrohr eingeleitet werden, wird stufenlos von 0 Nm³/h auf 1000 Nm³/h Kohlenmonoxid und stufenlos von 0 Nm³/h auf 800 Nm³/h Chlor erhöht. Nach 30 Minuten ergibt sich im Phosgen ein Gehalt an Kohlenmonoxid von 11 %. Die Temperatur im Mischrohr beträgt 20 °C und der Druck stellt sich unmittelbar auf 3,2 bar absolut ein. Das Mischgas aus Chlor und Kohlenmonoxid tritt anschließend in den auf 220 °C vorgewärmten Innenraum des Rohrbündel-Phosgengenerators ein und verdrängt den inerten Stickstoff.

Die Reaktion zu Phosgen springt direkt an und ist stark exotherm. Die Reaktionswärme wird über einen Wärmeträgerölkreislauf (Dekalin) mittels Siedekühlung abgeführt. Die Temperatur des Phosgens in der Austrittsleitung des Generators liegt nach 10 Minuten bei 234 °C und der Druck liegt bei 2,55 bar absolut. An dieser Stelle hat das Phosgen während der Anfahrphase einen Restchlorgehalt > 1000 ppm (oberhalb des Messbereiches von 1000 ppm). Das so hergestellte Phosgen wird, wie in den allgemeinen Herstellungsbedingungen beschrieben, auf 113 °C heruntergekühlt und in den nachgeschalteten Rohrbündel-Phosgengenerator geleitet. Da die Temperatur des Reaktors zu Beginn nur 30 °C beträgt und die Eduktkonzentration im Reaktionsgas gering ist, dauert es ca. 5 Minuten bis die exotherme Reaktion startet. In dem nachgeschalteten zweiten Reaktor wird das sich im hergestellten Phosgen befindliche Chlor und CO wie in den allg. Herstellungsbedingungen beschrieben zu Phosgen umgesetzt. Im Austritt des 2. Phosgengenerators beträgt der Restchlorgehalt während der Anfahrphase > 1000 ppm (oberhalb des Messbereiches von 1000 ppm).

Das so hergestellte und im Phosgenverflüssiger kondensierte Phosgen hat während der Anfahrphase einen erhöhten Chlorgehalt, da das Chlor teilweise im Phosgenverflüssiger mitkondensiert. Die Menge an Chlor in Phosgen verdünnt sich kontinuierlich durch nachgeschobenes Phosgen im Laufe eines Tages auf < 50 ppm Chlor im Endprodukt Phosgen. Der Einsatz von diesem mit Chlor verunreinigtem Phosgen führt für 18 Stunden zu Farbproblemen bei der Phosgenierung von MDA zum Endprodukt MDI. Die Farbzahl des MDI liegt bei > 0,200 und in der Spitze bei 0,220 (Gelbwert).

### Beispiel 4 (erfindungsgemäß) Heißfahrweise: beim Aufheizen der Generatoren CO fahren.

Eine Phosgen-Anlage wird angefahren, indem in ein inertisiertes Mischrohr um 10 Minuten zeitversetzt Kohlenmonoxid und Chlor gefahren werden. Die Menge der beiden Einsatzstoffe, die während der Anfahrzeit t von 30 Minuten, für Chlor entsprechend nur 20 Minuten, in das Mischrohr eingeleitet werden, wird stufenlos von 0 Nm³/h auf 1000 Nm³/h Kohlenmonoxid und stufenlos von 0 Nm³/h auf 800 Nm³/h Chlor, hochgezogen. Nach 30 Minuten ergibt sich im Phosgen ein Gehalt an Kohlenmonoxid von 11 %. Die Temperatur im Mischrohr beträgt 20 °C und der Druck stellt sich unmittelbar auf 3,2 bar absolut ein. Das Mischgas aus Chlor und Kohlenmonoxid tritt anschließend in den auf 220 °C vorgewärmten Innenraum des Rohrbündel-Phosgengenerators ein und verdrängt den inerten Stickstoff.

Die Reaktion zu Phosgen springt direkt an und ist stark exotherm. Die Reaktionswärme wird über einen Wärmeträgerölkreislauf (Dekalin) mittels Siedekühlung abgeführt. Die Temperatur des Phosgens in der Austrittsleitung des Generators liegt nach 10 Minuten bei 234 °C und der Druck liegt bei 2,55 bar absolut. An dieser Stelle hat das Phosgen während der Anfahrphase einen Restchlorgehalt 100-800 ppm. Das so hergestellte Phosgen wird, wie in den allgemeinen Herstellungsbedingungen beschrieben, auf 113 °C heruntergekühlt. In dem nachgeschalteten Rohrbündel-Phosgengenerator reagiert das restliche Chlor unter dem CO Überschuss zu Phosgen.

Das so hergestellte Phosgen wird, wie in den allgemeinen Herstellungsbedingungen beschrieben, kondensiert und in der Phosgenabsorption gesammelt. Nach 60 Minuten werden diese Mengenströme auf die gewünschte Solllast erhöht. Das Hochfahren der Anlage kann manuell oder mit einer Anfahrautomatik gestaltet werden. Die Anlage wird jeweils so schnell wie möglich auf die Solllast hochgefahren, wobei das während der Anfahrphase entstehende Phosgen einen Restchlorgehalt von 25 ppm hat. Diese Messung wird in der Austrittsleitung des Generators durchgeführt. Der Einsatz von diesem Phosgen, das nur mit Spuren an Chlor verunreinigt ist, führt in der Phosgenierung von MDA zu einem annähernd farblosen Endprodukt MDI mit einer Farbzahl von 0,170 (Gelbwert).

**Tabelle 1: Gegenüberstellung der Ergebnisse aus den Beispielen**

| **Beispiel** | **Chlor in Phosgen [ppm]** | **Farbzahl im MDI [Gelbwert]** | |
|---|---|---|---|
| **1** (Vergleich) | > 1000 | 0,240 | |
| **2** (erfindungsgemäß) | 22 | 0,160 | |
| **3** (Vergleich) | > 1000 | 0,220 | |
| **4** (erfindungsgemäß) | 25 | 0,170 | |

Wie die Beispiele zeigen, entsteht erst bei erfindungsgemäßem Anfahren des Phosgengenerators Phosgen mit geringen Chlorgehalten, das die Herstellung von nahezu farblosem MDI erlaubt.

## Patentansprüche

1. Verfahren zum Betreiben eines Phosgengenerators zur Herstellung von Phosgen durch Umsetzung von Kohlenmonoxid mit Chlor in der Gasphase an einem Aktivkohle-Katalysator, der in einem Reaktionsraum angeordnet ist, bei dem die Phosgenherstellung nach einer vorgebbaren Betriebsdauer durch Abfahren des Phosgengenerators über eine Abfahrdauer zumindest zeitweise unterbrochen und nach einer vorgebbaren Stillstandzeit durch Anfahren des Phosgengenerators über eine Anfahrzeit wieder aufgenommen wird,
**dadurch gekennzeichnet, dass**
der Aktivkohle-Katalysator vor dem Anfahren des Phosgengenerators durch Zugabe von Kohlenmonoxid soweit von Chlor befreit wird, dass während der Anfahrzeit eine Höchstkonzentration von Chlor im Gasstrom unmittelbar stromabwärts des Reaktionsraumes von 1000 ppmv nicht überschritten wird.

2. Verfahren nach Anspruch 1, **dadurch gekennzeichnet, dass** eine Höchstkonzentration von Chlor im Gasstrom unmittelbar stromabwärts des Reaktionsraumes von 100 ppmv nicht überschritten wird, bevorzugt von 50 ppmv, insbesondere von 10 ppmv.

3. Verfahren nach Anspruch 1 oder 2, **dadurch gekennzeichnet, dass** zur Einhaltung der Höchstkonzentration von Chlor während des Abfahrens des Phosgengenerators die Chlorzufuhr über die Abfahrdauer reduziert oder unmittelbar unterbrochen wird, wobei die Zufuhr an Kohlenmonoxid so lange aufrechterhalten wird, bis die Höchstkonzentration von Chlor erreicht oder unterschritten ist.

4. Verfahren nach Anspruch 3, **dadurch gekennzeichnet, dass** der Aktivkohlekatalysator während der Abfahrdauer auf einer Temperatur von 60 °C bis 140 °C gehalten wird.

5. Verfahren nach einem der vorstehenden Ansprüche, **dadurch gekennzeichnet, dass** sich der Phosgengenerator in der Stillstandzeit befindet und vor dessen Anfahren die Kohlenmonoxid Zufuhr gestartet wird, sowie
a) der Aktivkohle-Katalysator auf eine Temperatur von 60 °C bis 140 °C erwärmt wird und/ oder
b) der Kohlenmonoxid Gasstrom auf eine Temperatur von 130 °C bis 250 °C erwärmt wird,
sodass das noch aus dem vorigen Produktionszyklus auf dem Aktivkohle-Katalysator und/ oder im Reaktionsraum vorhandene Chlor bis zum Erreichen oder Unterschreiten der Höchstkonzentration abreagiert.

6. Verfahren nach Anspruch 5, **dadurch gekennzeichnet, dass** nach Erreichen oder Unterschreiten der Höchstkonzentration an Chlor der Aktivkohle-Katalysator auf einer Temperatur von wenigstens 140 °C, insbesondere wenigstens 180 °C erwärmt wird, bevor die Chlorzufuhr gestartet wird.

7. Verfahren nach einem der vorstehenden Ansprüche, **dadurch gekennzeichnet, dass** die zum Erreichen oder Unterschreiten der Höchstkonzentration an Chlor eingesetzte Menge an Kohlenmonoxid mindestens 40 Normkubikmeter pro Tonne Aktivkohle-Katalysator im Reaktionsraum beträgt, insbesondere mindestens 60 Normkubikmeter, bevorzugt mindestens 80 Normkubikmeter.

8. Verfahren nach einem der vorstehenden Ansprüche, **dadurch gekennzeichnet, dass** der Beginn des Anfahrens des Phosgengenerators durch den Start der Chlorzufuhr definiert ist.

9. Verfahren nach einem der vorstehenden Ansprüche, **dadurch gekennzeichnet, dass** die Chlorzufuhr über die Anfahrzeit bis zu einem gewünschten Endwert gesteigert wird, wobei die Steigerung insbesondere stufenweise erfolgt und die Stufen vorzugsweise 25%, 50%, 75% und 100% des gewünschten Endwerts betragen und/ oder wobei die stufenweise Steigerung bevorzugt in gleichen Zeitintervallen erfolgt.

10. Verfahren nach einem der vorstehenden Ansprüche, **dadurch gekennzeichnet, dass** die Volumenkonzentration an Chlor UV-spektrometrisch bestimmt wird.

11. Verfahren nach einem der vorstehenden Ansprüche, **dadurch gekennzeichnet, dass** während der Anfahrzeit und der Betriebsdauer ein molarer Überschuss an CO gegenüber Chlor von 2 Mol-% bis 20 Mol-% eingestellt wird.

12. Verwendung von nach einem der Ansprüche 1 bis 11 gewonnenem Phosgen in der Herstellung von Polycarbonat und/oder Isocyanaten.
